# EUROPEAN PATENT SPECIFICATION

(11) **EP 4 574 113 B1**
(45) Date of publication and mention of the grant of the patent: **27.05.2026**
(21) Application number: 23219064.5
(22) Date of filing: 21.12.2023
(51) Int. Cl.: A61F 13/00, A61F 13/0203, A61F 13/0246, A61F 13/02

(54) **A PRINTED MEDICAL DRESSING**
BEDRUCKTER MEDIZINISCHER VERBAND
PANSEMENT MÉDICAL IMPRIMÉ

(43) Date of publication of application: 25.06.2025
(73) Proprietor: Mölnlycke Health Care AB, 431 21 Mölndal (SE)
(72) Inventor: MADLUNG, Peter, 416 57 GÖTEBORG (SE)
(74) Representative: Kransell & Wennborg KB

(56) References cited:
- WO-A1-2023/051894
- US-A1- 2002 040 202
- US-A1- 2015 065 937
- US-A1- 2021 393 443
- US-A1- 2023 029 178
- US-A1- 2023 225 906

## Description

### TECHNICAL FIELD

The present disclosure generally relates to a medical dressing comprising a transparent or translucent backing layer, an adhesive skin-contact layer, and an absorbent pad arranged between the backing layer and the adhesive skin-contact layer. The medical dressing comprises a printed marking. The present disclosure also relates to method for manufacturing the medical dressing.

### BACKGROUND

Adhesive medical dressings are frequently used in wound care, both for the purpose of treating wounds and scars and for the purpose of preventing these from occurring in the first place.

Examples of adhesive medical dressings are described in US 2023/0225906 A1, US 2023/0029178 A1, US 2002/0040202 A1, US 2015/0065937 A1, US 2021/0393443 A1 and WO 2023/051894 A1.

An adhesive medical dressing typically comprises an adhesive skin-contact layer arranged to contact the skin or wound of a patient, as well as a top layer, often referred to as a backing layer. In many cases, particularly when the dressing is to be applied to moderate or highly exuding wounds, the dressing further comprises a wound pad arranged between the backing layer and the adhesive skin-contact layer. To protect the dressing from contamination, a release liner is applied to the adhesive skin-contact layer.

Adhesive medical dressings are typically individually packed in pouches, which keep the dressings sterile prior to use. A plurality of pouches housing the dressings are generally contained and stored in an outer package.

The pouch and the outer package typically contain a variety of product-identifying information. For example, lot lumbers, batch numbers, and dressing-specific information may be printed on the pouch. Furthermore, instructions relating to a correct use or application of the dressing may be provided in an instruction manual ("Instructions for use", IFU), which comes with the package. Such information may also be printed on the outer package.

However, in many cases, usage- or product-related information (as provided in e.g. an IFU or on the outer package) is not present in the actual care situation and may easily become lost or dislocated. Also, once the dressing has been applied to the wound or skin of a patient, the pouch is typically discarded. In this regard, it becomes very difficult (in general impossible) to trace an applied medical dressing back to the manufacturing stage e.g. for potential product recall or regulatory purposes.

Another problem, in the field of wound dressings, is counterfeiting. Counterfeiting is a fraudulent imitation of the product or brand. A counterfeit product is typically formed from cheaper materials and may thus be associated with poor quality and/or health and safety issues. From a consumer-point-of view, it may be very difficult to distinguish a product from a trusted supplier from a counterfeit product.

**In** view of the scenarios described above, it would be desirable to print product-identifying markings on the actual dressing such that the markings are visibly observable even when the medical dressing has been applied to the skin or wound of a patient.

A challenge associated with printing directly on the medical dressing is the risk of inks or dyes interfering with the skin or wound of a patient. To overcome this problem, attempts have been made to print markings (e.g. instructions or graphical elements) onto the release liner of the medical dressing. However, the release liner is peeled off prior to application of the dressing onto the skin or the wound. In other words, the problems outlined hereinabove remain unresolved.

In view of the above, there is need to provide a safe and reliable printed medical dressing, wherein the printed marking is visibly observable even when the medical dressing has been applied to the skin or wound of a patient. Such a dressing should avoid and/or reduce the risk of the ink or dye interfering with the wound or skin.

### SUMMARY

In view of the above-mentioned problems, it is an object of the present disclosure to provide improvements in the field of printed medical dressings.

According to a first aspect, there is provided a medical dressing comprising a transparent or translucent backing layer, an adhesive skin-contact layer, and an absorbent pad arranged between the backing layer and the adhesive skin-contact layer, wherein the absorbent pad is defined by peripheral edges; the backing layer and the adhesive skin-contact layer being arranged to extend beyond the peripheral edges to form a border portion surrounding the absorbent pad, wherein the adhesive skin-contact layer comprises a plurality of perforations in the area underlying the absorbent pad, wherein the medical dressing comprises at least one printed marking arranged between the backing layer and the adhesive skin-contact layer in the border portion and wherein the adhesive skin-contact layer is non-perforated in the area underlying the printed marking(s).

The present disclosure is based on the realization that the provision of printed markings between the backing layer and the adhesive skin-contact layer prevents the risk of the colored ink or dye from interfering with the wound. The printed marking is arranged in the border portion; i.e. remotely from the absorbent pad. This is beneficial since the absorbent pad is often arranged directly above the wound. Since the adhesive wound contact layer is perforated in the area underlying the absorbent pad, each of the layers overlying the adhesive skin-contact layer in this area is in fluid communication with the wound site. The provision of e.g. a colored ink or dye in this region may thus be associated with risks of ink or dye elution to the wound site.

However, the provision of the printed marking(s) in the border portion between the backing layer (which is a continuous layer), and an a non-perforated part of the adhesive skin-contact layer secures that the printed marking(s) is encapsulated and shielded. The ink or dye is thereby prevented from interfering with any of the other layers of the dressings which are in fluid communication with the wound site. Accordingly, the dressing may be safely applied to the skin or wound of a patient without risking elution of ink to the wound site.

Furthermore, the encapsulation of the printed marking in this region also protects the printed marking from becoming damaged due to wear and tear. Printing on the outermost surface of the backing layer enhances the risk of the printed marking becoming chafed and damaged during use.

The backing layer of the dressing is transparent or translucent such that the printed marking is visibly observable when the patient is wearing the medical dressing. This is beneficial for the purpose of conveying product-identifying and/or usage-specific information to the patient and/or his/her caregiver. It may also be beneficial for the purpose of yielding a more visually appealing impression.

Preferably, the adhesive skin contact layer is non-perforated in the entire area forming the border portion.

Accordingly, the entire border portion is void of perforations. The encapsulation and shielding effect of the printed marking(s) is thereby improved.

Furthermore, a non-perforated border portion is also beneficial for the purpose of enhancing the adherence of the dressing to the skin, such that the dressing may stay on the skin for prolonged periods.

The perforations underlying the absorbent pad secure an efficient liquid acquisition, and liquid handling in the area of the dressing where this is desirable, i.e. in the absorbent pad.

The backing layer has a first side facing the adhesive skin-contact layer, and an opposing second side, wherein the at least one printed marking may be arranged on the first side of the backing layer.

Furthermore, the adhesive skin-contact layer has a first side facing the backing layer, and an opposing second side, wherein the at least one printed marking may be arranged on the first side of the adhesive skin-contact layer.

It is equally conceivable to print on the first side of the backing layer or on the first side of the adhesive skin-contact layer. In both of these scenarios, the printed marking will become encapsulated in the border portion.

The first side of the backing layer and/or the first side of the adhesive skin-contact layer may be provided with an adhesive to secure the attachment of these two layers in the border portion of the dressing.

The printed marking may be provided either before or after the application of such an adhesive.

Accordingly, it is conceivable that the printed marking is provided on the first side of the backing layer or the first side of the adhesive skin-contact layer which contains an applied adhesive.

The adhesive-skin contact layer may comprise a polymeric film and an adhesive silicone gel adhesive, wherein the silicone gel adhesive is arranged to contact the skin of a wearer during use.

An adhesive skin-contact layer comprising a silicone gel adhesive is skin-friendly and easy to remove without causing trauma. It is sufficiently adherent to skin such that the dressing stays in place, but is configured to maintain its adherence with repeated removal and re-application.

The polymeric film yields rigidity to the border portion, which is typically thin and flimsy, and thus facilitates the attachment (and detachment) of the dressing to the skin.

In exemplary embodiments, the adhesive skin-contact layer may be translucent or transparent.

A transparent or translucent adhesive skin-contact layer enhances the visibility of the printed marking.

Typically, the at least one printed marking is formed by a colored ink.

The present disclosure is not limited to a specific colored ink, but any ink is conceivable. There are no specific safety concerns associated with the colored ink per se since the ink will be encapsulated and shielded from the wound site, the surroundings, and also from the layer(s) of the wound pad. It should be noted that a wound dressing is subject to stringent regulatory provisions so any material that is at risk of interfering with the wound site is to be avoided. However, since the colored ink is arranged in a manner that eliminates ink elution risks, the dressing can be safely applied to the patient.

Preferably, the medical dressing is void of printed markings formed by colored ink(s) in the area of the medical dressing where the absorbent pad is arranged.

As mentioned hereinbefore, the adhesive skin-contact layer is perforated in the area underlying the absorbent pad. This is beneficial for the purpose of wound exudate absorption, and for efficient liquid handling of the medical dressing. However, it is also associated with ink elution risks as outlined hereinabove. In this regard, it is beneficial to avoid colored inks in the vicinity of the wound pad.

It is, of course, conceivable to apply a printed marking in the area of the medical dressing where the absorbent pad is arranged if such a printed marking is "safe to use". For example, printed markings which do not rely on colored inks are conceivable. Such markings may e.g. be provided by means of UV laser printing. This printing technique does not suffer from problems with ink elution.

In exemplary embodiments, the at least one printed marking may be a graphical element and/or a text element.

The graphical element may be a symbol, a figure, an image, a photograph or a visual element. The graphical element may be associated with the brand or the product, or it may be used to convey information, e.g. product-identifying information or usage-specific information about the medical dressing.

The text element may e.g. be a brand name, sub-brand, a product descriptor or a size indication. It is also conceivable that the printed marking indicates a correct use or application of the medical dressing.

As mentioned hereinbefore, usage-specific information are often contained in an IFU, which often becomes dislocated after the dressing has been applied to the skin of a patient. If such instructions are provided directly on the dressing, the caregivers are offered a clear and direct guidance of how to use and/or apply the dressing correctly.

Preferably, the printed marking carries product-identifying information.

For example, the printed marking may be a batch number, a production lot number, and/or a country indication.

Such markings are beneficial for regulatory purposes, anti-counterfeiting and potential product recall purposes and enables authentication of the product. Accordingly, each dressing may be tracked and traced, e.g. back to the earliest steps of manufacturing. By printing a batch number or a production lot number directly on the dressing, the machine, the materials used, as well as the precise manufacturing conditions may be easily tracked, which significantly facilitates the efforts associated with potential product recall issues.

The provision of a printed country indication is also useful for preventing illegal imports; i.e. branded products being imported into a market and sold there without the trademark owner's consent in that market or country. The country indication may be the country of manufacturing or the country of commercialization.

In exemplary embodiments, the at least one printed marking is a machine-readable tag, preferably a bar code or a QR code.

The bar code or QR code may encode an internet or web address containing technical and/or usage-specific information related to the medical dressing.

Hence, information about the medical dressing may be accessed easily. To date, such information is typically present in an IFU or on the pouch or package of the dressings, which is typically not accessible after the dressing has been applied to a patient.

It is also conceivable that the information about the batch number, production lot number or country indication is encoded by the QR code or bar code.

According to another aspect, there is provided a package, preferably a pouch, comprising the medical dressing as described hereinbefore, wherein the package comprises at least one printed marking corresponding to the at least one printed marking on the medical dressing.

According to yet another aspect, there is provided a method for manufacturing a medical dressing comprising:
a) providing a transparent or translucent backing layer having a first side and an opposing second side;
b) arranging an absorbent pad on the first side of the backing layer; the absorbent pad being defined by peripheral edges;
c) arranging an adhesive skin-contact layer onto the absorbent pad; the adhesive skin-contact layer having a first side facing the backing layer, and an opposing, second side, wherein the backing layer and the adhesive skin-contact layer are arranged to extend beyond the peripheral edges of the absorbent pad to form a border portion surrounding the absorbent pad, and wherein the adhesive skin-contact layer comprises a plurality of perforations in the area underlying the absorbent pad, wherein
the method further comprises a step of providing at least one printed marking between the backing layer and the adhesive skin-contact layer in the border portion, and wherein the adhesive skin-contact layer is non-perforated in the area underlying the printed marking(s)

As mentioned hereinbefore, the printed marking is provided either on the first side of the backing layer or on the first side of the adhesive skin-contact layer.

In exemplary embodiments, the printed marking is provided on the first side of the backing layer, and wherein the step of providing the printed marking is performed after the step b) of arranging an absorbent pad on the first side of the backing layer.

Alternatively, the printed marking is provided on the first side of the adhesive skin-contact layer, and wherein the step of providing the printed marking is performed prior to the step c) of arranging the adhesive skin-contact layer on the absorbent pad.

Preferably, the step of providing a printed marking is performed as an in-line step in the method.

The method is preferably an automated or semi-automated process.

Accordingly, the method may be carried out in an efficient and quick manner and is suitable for large-scale production.

Further features of, and advantages with, the present disclosure will become apparent when studying the appended claims and the following description. The skilled addressee realizes that different features of the present disclosure may be combined to create embodiments other than those described in the following, without departing from the scope of the present disclosure. The invention is defined by the appended claims.

### BRIEF DESCRIPTION OF DRAWINGS

The various aspects of the present disclosure, including its particular features and advantages, will be readily understood from the following detailed description and the accompanying drawings, in which:
Figure 1 is a split view of a medical dressing comprising a printed marking according to an exemplary embodiment of the present disclosure.
Figure 2a illustrates a top-view of a medical dressing provided with printed markings in the form of a QR code and a batch number according to an exemplary embodiment of the present disclosure.
Figure 2b illustrates a bottom-view of the medical dressing in figure 2a, as seen from the adhesive skin-contact layer.
Figure 3 schematically illustrates the equipment and the process steps in the method of providing a medical dressing according to an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

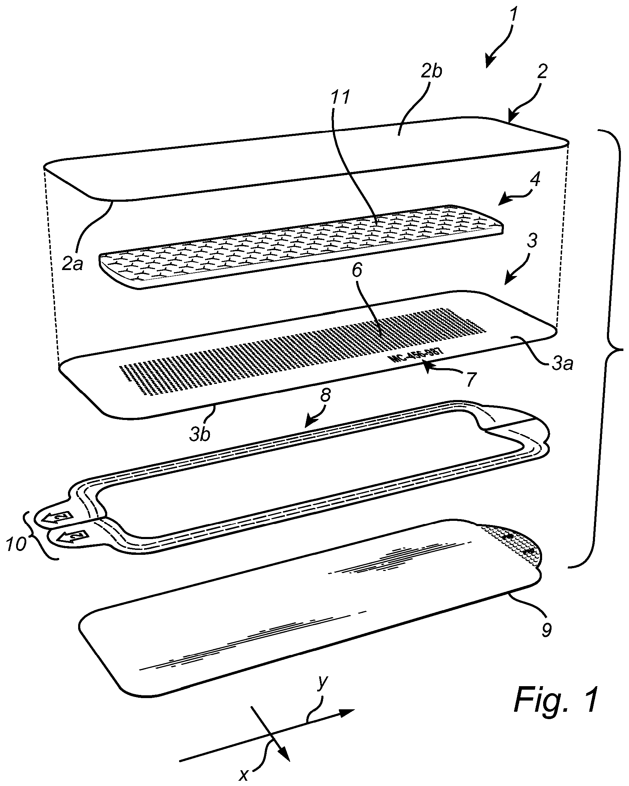

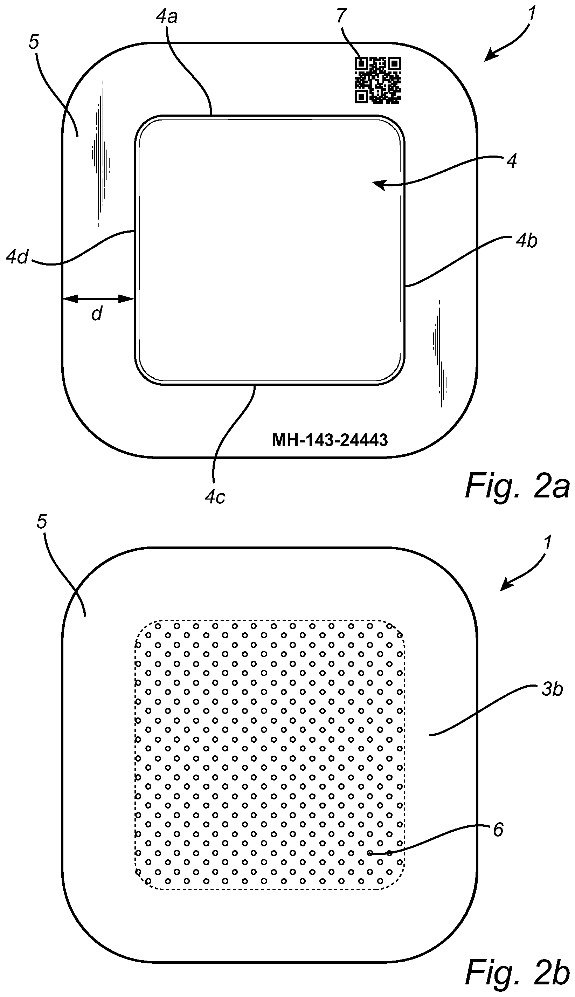

A medical dressing is schematically illustrated in figure 1 and figures 2a-b. The medical dressing 1 comprises a transparent or translucent backing layer 2, an adhesive skin-contact layer 3, and an absorbent pad 4 arranged between the backing layer 2 and the adhesive skin-contact layer 3, wherein the absorbent pad 4 is defined by peripheral edges 4a-d (see figure 2); the backing layer 2 and the adhesive skin-contact layer 3 being arranged to extend beyond the peripheral edges 4a-d to form a border portion 5 surrounding the absorbent pad 4, wherein the adhesive skin-contact layer 3 comprises a plurality of perforations 6 in the area underlying the absorbent pad 4, wherein the medical dressing 1 comprises at least one printed marking 7, wherein the printed marking(s) 7 is arranged between the backing layer 2 and the adhesive skin-contact layer 3 in the border portion 5, and wherein the adhesive skin-contact layer 3 is non-perforated in the area underlying the printed marking(s) 7.

As illustrated in figure 1 and in figure 2b, the adhesive adhesive-skin contact layer 3 comprises a perforated area and a non-perforated area. The perforated area is arranged in the area of the adhesive skin-contact layer 3 underlying the absorbent pad 4.

The area of the adhesive-skin contact layer 3 forming the border portion 5 (see figures 2b) is preferably non-perforated. Hence, the entire border portion 5 is void of perforations. This is beneficial to improve the adhesion to the skin in the border of the dressing such that the stay-on ability of the dressing is improved. It is furthermore beneficial to secure that the printed marking(s) 7 is encapsulated and prevented from eluting.

The entire area of the adhesive-skin contact layer 3 forming the border portion 5 need not be void of perforations. For the purpose of encapsulating the printed marking(s) 7 and shield it from interference with the wound site or the dressing layers in fluid communication with the wound site, it is conceivable that the adhesive skin-contact layer 3 is non-perforated in the area underlying the printed marking(s) 7.

The size of "the area underlying the printed marking(s)" is preferably at least two times larger, preferably at least four times larger than the size of the printed marking(s) 7.

For example, the adhesive skin contact layer 3 may be non-perforated in at least 50 % of the area forming the border portion 5, preferably in at least 75 % of the area forming the border portion 5.

In preferred embodiments, the adhesive skin contact layer 3 is non-perforated the entire area forming the border portion 5.

The perforations 6 in the adhesive skin-contact layer 3 arranged underneath the absorbent pad 4 improve the absorption of wound exudate and liquid handling of the dressing, and are therefore arranged in the area where absorption takes place.

The perforations may be of various shapes and sizes. Preferably, the perforations are arranged in a predetermined, regular pattern. The perforations may have a diameter of from 0.5 mm to 10 mm, e.g. from 1 to 5 mm, preferably from 1 to 2 mm.

The absorbent pad 4 is defined by peripheral edges. In figure 1, the medical dressing 1 is rectangular. In figure 2, the medical dressing is square shaped. However, the medical dressing of the present disclosure is not limited to a particular shape, but any shape is conceivable, such as oval, round etc. It is also conceivable that the medical dressing has a shape adapted to fit with the sacrum or heel of a wearer. In such cases, the medical dressing may comprise a one or several protruding portions.

Accordingly, the peripheral edges of the absorbent pad 4 may be straight (as illustrated in figures 1 and 2) or curved.

The backing layer 2 may be attached to the adhesive skin-contact layer 3 in the area of the border portion 5. For example, the backing layer 2 may be attached to the adhesive skin-contact layer 3 by an adhesive, e.g. a polyacrylate adhesive. Heat lamination of the backing layer 2 and adhesive skin-contact layer is also conceivable.

The backing layer 2 and the adhesive skin-contact layer 3 are co-extensive; i.e. they have the same length and width.

The size of the absorbent pad 4 is smaller than the size of the backing layer 2 and the adhesive skin-contact layer 3.

The border portion 5 surrounds the absorbent pad. In other words, the border portion extends a distance, d, beyond each peripheral edge of the absorbent pad 4 (see figure 2a).

The distance, d, may be the same or it may differ. For example, if the dressing has a more "complex" shape, as is the case with e.g. heel or sacral dressings, some parts of the medical dressing may have a larger border portion. Accordingly, the distance, d, may vary along the peripheral edges of the medical dressing. Alternatively, the distance, d, is the same around the whole periphery of the absorbent pad.

In order to secure a firm attachment to the skin and to enable printing, the border portion may have a width of at least 10 mm, e.g. from 10 to 50 mm.

A smaller sized dressing may have a smaller border portion than a larger sized dressing.

The width of the border portion means the distance, d, from a peripheral edge of the absorbent pad to a peripheral edge of the border portion (i.e. a peripheral edge of the medical dressing). The width of the border portion is usually measured using the shortest distance from a peripheral edge of the absorbent pad to a peripheral edge of the border portion (i.e. a peripheral edge of the medical dressing).

The maximum width; i.e. maximum lateral (x) extension of the dressing may be from 35 to 250 mm.

The maximum length; i.e. maximum longitudinal (y) extension of the dressing may be from 50 to 450 mm.

The medical dressing 1 typically further comprises a release liner detachably attached to the adhesive skin-contact layer.

The release liner may comprise one or a plurality of release liner portion, and is configured to cover the entire surface of the adhesive skin-contact layer 3.

In figure 1, the release liner is formed by a first frame release liner portion 8, and a second release liner portion 9. The frame release liner portion 8 is arranged between the adhesive skin-contact layer 3 and the second release liner portion 9. The frame release liner portion 8 may overlap with the printed marking(s) 7.

The present disclosure is by no means limited to this construction. However, this configuration is beneficial for facilitating application of the medical dressing to the skin. The border portion of the medical dressing is typically very thin and prone to wrinkle formation. The dressing may be applied to the skin in a controlled manner. The second release liner portion 9 may gradually be removed while simultaneously anchoring the dressing to the skin. **In** a next step, the first frame release portion 8 is removed, by grasping one of the tabs 10, while simultaneously anchoring the border portion 5 of the dressing to the skin.

The "backing layer" is the top layer of the dressing; i.e. the outermost layer of the dressing. The backing layer is a continuous layer. The backing layer is void of perforations and incisions. The backing layer protects the layers of the dressing from potential contaminants.

The backing layer is desirably a thin and pliable layer such that it can stretch and conform to the movement of a wearer. The backing layer may be a polymeric film. Suitably, the backing layer comprises a polyurethane film. The backing layer may also be a laminate of a nonwoven layer and at least one polyurethane film. The thickness of the backing layer may be in the range of from 15 to 50 µm, preferably from 20 to 40 µm.

The backing layer is translucent or transparent. Hence, light is allowed to pass through the backing layer such that the printed marking(s) 7 can be visibly observed. Preferably, the backing layer 2 is transparent.

As illustrated in figure 1 the backing layer 2 has a first side 2a facing the adhesive skin-contact layer 3, and an opposing second side 2b. The at least one printed marking 7 may be arranged on the first side 2a of the backing layer 2.

In embodiments where the backing layer 2 and the adhesive skin-contact layer are adhesively attached, the printed marking(s) may be provided either directly on the first side 2a of the backing layer 2 or on the adhesive provided on the first side 2a.

The "adhesive skin-contact layer" is configured to detachably adhere the dressing to a dermal surface. In other words, the adhesive skin-contact layer is configured to contact the skin or the wound of a wearer. This layer may also be referred to as a "wound contact layer".

The adhesive skin-contact layer 3 has a first side 3a facing the backing layer 2, and an opposing second side 3b. The at least one printed marking 7 may be arranged on the first side 3a of the adhesive skin-contact layer (as illustrated in figure 1).

The adhesive-skin contact layer 3 may comprise a polymeric film and an adhesive silicone gel; the silicone gel being arranged to contact the skin of a wearer during use.

An adhesive skin-contact layer comprising a silicone gel is skin-friendly and easy to remove without causing trauma. It is sufficiently adherent to skin such that the dressing stays in place, but is configured to maintain its adherence with repeated removal and re-application.

Examples of suitable silicone gels for use in the adhesive skin-contact layer include the two component RTV systems, such as Q72218 (Dow Corning), and SilGel 612 (Wacker Chemie AG), as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the adhesive skin-contact layer is typically at least 20 µm. The thickness of the adhesive skin-contact layer may be from 50 to 200 µm.

The polymeric film may be a breathable film and may comprise e.g. polyethylene, polyamide, polyester or polyurethane. Preferably, the polymer-based film comprises polyurethane. The thickness of the polymeric film may be from 15 to 100 µm, e.g. from 20 to 80 µm, preferably from 20 to 60 µm.

The adhesive skin-contact layer 3 may be translucent or transparent to improve the visibility of the printed marking(s) 7.

Hence, the printed marking(s) may be visibly observable when viewed from the bottom-side of medical dressing, i.e. from the second side 3b of the adhesive skin contact layer 3. In figure 2b, the medical dressing is seen from the bottom-side of the medical dressing. For ease of illustration, the printed marking(s) are not shown in this view.

The printed marking(s) 7 is typically formed by a colored ink.

Any colored ink may be used. The ink may be water based or solvent based; i.e. the ink may comprise an organic solvent, such as an alcohol, ester etc. that can dissolve the pigment, resin and potentially other additives. For example, a flexographic ink may be used.

Preferably, the medical dressing 1 is void of printed markings in the area of the medical dressing 1 where the absorbent pad 4 is arranged.

More particularly, the medical dressing 1 is void of printed marking(s) formed by a colored ink in the area of the medical dressing 1 where the absorbent pad 4 is arranged.

This is to avoid the ink interfering with the wound dressing layers which are in fluid communication with the wound site.

As mentioned hereinbefore, printed marking(s) may be provided on e.g. the backing layer in the area overlying the absorbent pad 4. However, such printed marking(s) then need to free from conventional colored inks. Such printed marking(s) could e.g. be formed by printing with UV laser, wherein no colored ink is utilized.

The at least one printed marking 7 may be a graphical element and/or a text element.

As used herein, the term "graphical element" means a symbol, a figure, an image, a photograph, a logotype, a shape, a pattern, and/or a visual element. The graphical element may be associated with the brand, or it may be used to convey information, e.g. product-identifying information or usage-specific about the medical dressing 1.

The "text element" may be a brand name, sub-brand, a product descriptor, an alphabet letter, a number, a size indication, and/or a combination of letters and numbers.

It is also conceivable that the at least one printed marking 7 may indicate a correct use or application of the medical dressing 1.

Preferably, the printed marking(s) 7 carries product-identifying information.

The at least one printed marking 7 may be a batch number, a production lot number, or a country indication.

A dressing comprising a printed marking in the form of a batch number is illustrated in figure 1 and figure 2b.

Such markings are beneficial for regulatory purposes, anti-counterfeiting and potential product recall purposes and improves the traceability and authentication of the product. Accordingly, each dressing may be tracked and traced back to the earliest steps of manufacturing.

To date, e.g. the lot number may be provided on the package comprising the dressing. However, the package is often discarded after application of the medical dressing. This renders the traceability of the dressing more difficult since a potential "problem" may be detected at a later stage in time.

It is also conceivable that the printed marking(s) comprises the lot sequence number; i.e. the exact sequence number in a lot of dressings. This is useful to enable an exact tracing of a potential error in the manufacturing chain.

A printed country indication is also useful for preventing illegal imports, which is a common problem in the field of advanced medical dressings. The country indication may be the country of manufacturing or the country of commercialization.

The at least one printed marking 7 may be a machine-readable tag, preferably a two-dimensional machine-readable tag such as a bar code a QR code.

Combinations of different printed markings are also possible such as a combination of e.g. a machine-readable tag and a graphical element. Optionally, combinations of different printed markings such as a machine-readable tag and a graphical element and/or a text element such as product-identifying information is possible, just to mention some combinations. It is also possible to have a plurality of the same printed markings such as two or more identical printed markings. This can be advantageous when printing machine-readable tags, since irregularities in the dressing material may cause a machine-readable tag to have a lower chance for generating successful readings. Hence having multiple identical machine-readable tags can be favorable. It can also reduce the requirements for a sophisticated synchronization of the printed marking with the non-perforated area of the adhesive skin contact layer.

A medical dressing 1 comprising a printed QR code and a batch number is e.g. illustrated in figure 2a.

The bar code or QR code may encode an internet or web address containing e.g. technical and/or usage-specific information related to the medical dressing.

It is also conceivable that information about the batch number, production lot number or country indication is encoded by the QR code or bar code.

In the context of the present disclosure, the "absorbent pad" 4 may comprise one or a plurality of pad-forming layers.

The absorbent pad 4 may e.g. comprise an absorbent polyurethane foam layer.

The absorbent pad 4 may further comprise a superabsorbent layer; i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad 4 may also comprise a liquid distribution layer. The liquid distribution layer may comprise any material having the ability to distribute the exudate in an efficient manner. For example, the liquid distribution layer may comprise a nonwoven material.

A layered pad construction prevents accumulation of body liquids close to the skin and improves the overall liquid handling of the medical dressing.

The absorbent pad 4 may comprise a plurality of incisions 11 extending through at least a part of the absorbent pad 4. The incisions 11 enhance the flexibility of the absorbent pad, which is beneficial to secure a prolonged stay-on ability.

In another aspect, there is provided a package, preferably a pouch, comprising the medical dressing 1 as described hereinbefore, wherein the package comprises at least one printed marking corresponding to the at least one printed marking 7 on the medical dressing 1.

The pouch may comprise a front layer and a back layer. The printed marking(s) may be provided on the back layer.

The front layer may e.g. comprise polyethylene or polyethylene terephthalate (PET). The front layer is typically transparent such that the medical dressing can be seen through the pouch.

The back layer may e.g. comprise paper.

According to yet another aspect, there is provided a method for manufacturing a medical dressing comprising:
a) providing a transparent or translucent backing layer 2 having a first side 2a and an opposing second side 2b;
b) arranging an absorbent pad 4 on the first side 2a of the backing layer 2; the absorbent pad 4 being defined by peripheral edges 4a-d;
c) arranging an adhesive skin-contact layer 3 onto the absorbent pad 4; the adhesive skin-contact layer having a first side 3a facing the backing layer 2, and an opposing, second side 3b, wherein the backing layer 2 and the adhesive skin-contact layer 3 are arranged to extend beyond the peripheral edges 4a-d of the absorbent pad 4 to form a border portion 5 surrounding the absorbent pad 4, and wherein the adhesive skin-contact layer 3 comprises a plurality of perforations 6 in the area underlying the absorbent pad 4, wherein
the method further comprises a step of providing at least one printed marking 7 between the backing layer 2 and the adhesive skin-contact layer 3 in the border portion 5, and wherein the adhesive skin-contact layer 3 is non-perforated in the area underlying the printed marking(s) 7.

The printed marking 7 may be provided either on the first side 2a of the backing layer 2 or on the first side 3a of the adhesive skin-contact layer 3.

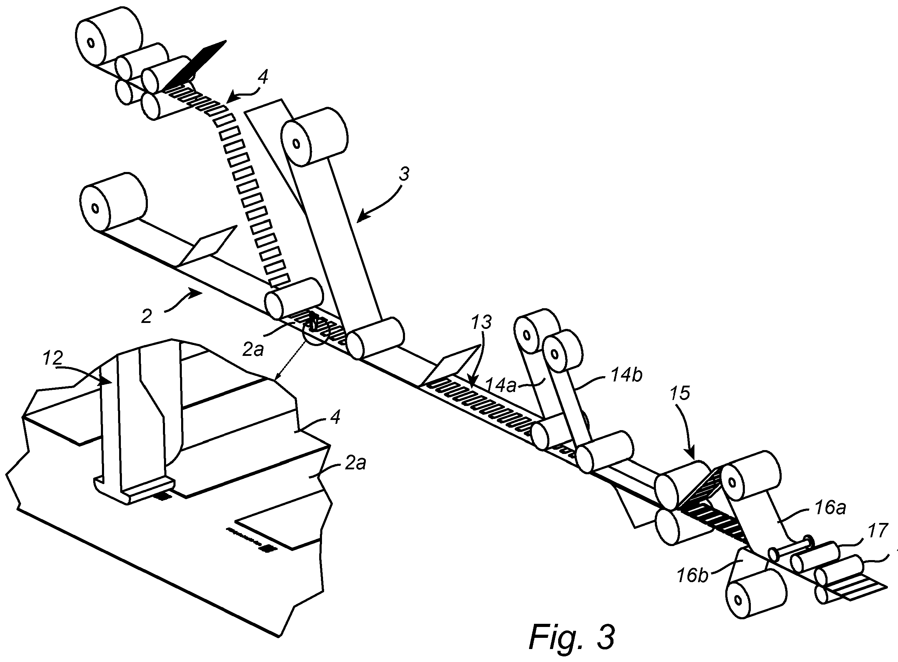

As illustrated in figure 3, absorbent pads 4 are arranged on a backing layer web 2. The first side of the backing layer web corresponds to the first side of the backing layer and is denoted 2a in figure 3. The absorbent pads 4 are arranged in contact with the first side 2a of the backing layer web 2.

**In** figure 3, the at least one printed marking 7 is provided on the first side 2a of the backing layer 2, and wherein the step of providing the printed marking 7 is performed after step b) of arranging an absorbent pad 4 on the first side 2a of the backing layer 2.

As mentioned hereinbefore, it is also conceivable that the printed marking 7 is provided on the first side 3a of the adhesive skin-contact layer 3, and wherein the step of providing the printed marking 7 is performed prior to the step c) of arranging the adhesive skin-contact layer on the absorbent pad 4.

The marking(s) 7 may be printed on the first side of the backing layer 2 or the adhesive skin-contact layer 3 by any conventional printing technique known in the art, including, but not limited to a gravure printing, a flexographic printing, an offset printing, thermal transfer printing, an ink jet printing and the like.

In figure 3, the printing device is denoted 12. The printing device may be piezo inkjet printer, a thermal inkjet printer, a continuous inkjet printer or a thermal transfer printer.

Subsequently, an adhesive skin contact-layer 3 is arranged on the absorbent pad. In figure 3, the adhesive skin-contact layer 3 is provided as an adhesive skin contact layer web. When the adhesive skin-contact layer web 3 has been applied, a wound dressing web assembly 13 is provided. In figure 3, the adhesive skin-contact layer web 3 is transparent so the absorbent pads 4 are visibly observable through the adhesive skin-contact layer web 3.

One or several release liner portion(s) 14a-b may then be applied onto the wound dressing web assembly 13. The release liner portion(s) 14a-b are applied onto the second side of the adhesive skin-contact layer 3. In figure 3, two release liner portions 14a-b are used.

The method may further comprise a step of cutting out individual wound dressings from the wound dressing web assembly.

A cutting tool is denoted 15 in figure 3.

The method is not limited to a specific cutting technique, but any means known to the skilled person may be used.

The method may further comprise a step of packaging the medical dressings.

A first packaging layer 16a may be arranged on a first surface of the dressing, and a second packaging layer 16b may be arranged on an opposing, second surface of the dressing.

The packaging layers 16a-b may subsequently be sealed by a sealing tool 17, and thereafter cut by a cutting tool 18.

The first 16a and second 16b form the front layer and back layer of the pouch, which houses the medical dressing.

The method may further comprise a step of sterilizing the pouch (housing the medical dressing 1), e.g. by ethylene oxide (EtO) sterilization. This sterilization method allows the medical dressing to be sterilized in the final package, as ethylene oxide permeates the sealed films and cartons used to package the dressings.

It is also conceivable that the medical dressing is sterilized prior to being packaged. Since the printed marking(s) 7 is encapsulated and shielded between the backing layer and a non-perforated portion of the adhesive skin-contact layer, the printed marking(s) 7 is protected during sterilization.

The various layers of the wound dressings may be attached to one another by conventional means, such as by means of adhesives.

Preferably, the step of providing a printed marking 7 is performed as an in-line step in the method.

The advantage of providing the printed marking(s) 7 in-line is that manufacturing parameters and be collected and connected with the actual product through the printed marking(s) 7.

Preferably, the method is an automated or semi-automated process.

Accordingly, the method may proceed without interruptions, and no manual steps are required.

Terms, definitions and embodiments of all aspects of the present disclosure apply mutatis mutandis to the other aspects of the present disclosure.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (1) comprising a transparent or translucent backing layer (2), an adhesive skin-contact layer (3), and an absorbent pad (4) arranged between said backing layer (2) and said adhesive skin-contact layer (3), wherein said absorbent pad (4) is defined by peripheral edges (4a-d); said backing layer (2) and said adhesive skin-contact layer (3) being arranged to extend beyond said peripheral edges (4a-d) to form a border portion (5) surrounding said absorbent pad (4), wherein said adhesive skin-contact layer (3) comprises a plurality of perforations (6) in the area underlying said absorbent pad (4), **characterized in that** said medical dressing (1) comprises at least one printed marking (7) arranged between said backing layer (2) and said adhesive skin-contact layer (3) in said border portion (5) and wherein said adhesive skin-contact layer (3) is non-perforated in the area underlying said printed marking(s) (7).

2. The medical dressing (1) according to claim 1, wherein said adhesive skin contact layer (3) is non-perforated in the area forming said border portion (5).

3. The medical dressing (1) according to claim 1 or claim 2, wherein said backing layer (2) has a first side (2a) facing said adhesive skin-contact layer (3), and an opposing second side (2b), wherein said at least one printed marking (7) is arranged on said first side (2a) of said backing layer (2).

4. The medical dressing (1) according to any one of claims 1-3, wherein said adhesive skin-contact layer (3) has a first side (3a) facing said backing layer (2), and an opposing second side (3b), wherein said at least one printed marking (7) is arranged on said first side (3a) of said adhesive skin-contact layer.

5. The medical dressing (1) according to any one of the preceding claims, wherein said adhesive-skin contact layer (3) comprises a polymeric film and an adhesive silicone gel; said silicone gel being arranged to contact the skin of a wearer during use.

6. The medical dressing (1) according to any one of the preceding claims, wherein said adhesive skin-contact layer (3) is translucent or transparent.

7. The medical dressing (1) according to any one of the preceding claims, wherein said at least one printed marking (7) is formed by a colored ink.

8. The medical dressing (1) according to claim 7, wherein said medical dressing (1) is void of said printed marking(s) in the area of the medical dressing (1) where said absorbent pad (4) is arranged.

9. The medical dressing (1) according to any one of the preceding claims, wherein said at least one printed marking (7) is a graphical element and/or a text element.

10. The medical dressing (1) according to any one of the preceding claims, wherein said at least one printed marking (7) is a batch number, a production lot number, or a country indication.

11. The medical dressing (1) according to any one of the preceding claims, wherein said at least one printed marking (7) is a machine-readable tag, preferably a bar code or a QR code.

12. A package, preferably a pouch, comprising the medical dressing (1) according to any one of claims 1-11, wherein said package comprises at least one printed marking corresponding to said at least one said printed marking (7) on said medical dressing (1).

13. A method for manufacturing a medical dressing comprising:
a) providing a transparent or translucent backing layer (2) having a first side (2a) and an opposing second side (2b);
b) arranging an absorbent pad (4) on said first side (2a) of said backing layer (2); said absorbent pad (4) being defined by peripheral edges (4a-d);
c) arranging an adhesive skin-contact layer (3) onto said absorbent pad (4);
said adhesive skin-contact layer having a first side (3a) facing said backing layer (2), and an opposing, second side (3b), wherein said backing layer (2) and said adhesive skin-contact layer (3) are arranged to extend beyond said peripheral edges (4a-d) of said absorbent pad (4) to form a border portion (5) surrounding said absorbent pad (4), and wherein said adhesive skin-contact layer (3) comprises a plurality of perforations (6) in the area underlying said absorbent pad (4), **characterized in that**
said method further comprises a step of providing at least one printed marking (7) between said backing layer (2) and said adhesive skin-contact layer (3) in said border portion (5), and wherein said adhesive skin-contact layer (3) is non-perforated in the area underlying said printed marking(s) (7).

14. The method according to claim 13, wherein said printed marking (7) is provided on the first side (2a) of said backing layer (2) or on the first side (3a) of said adhesive skin-contact layer (3).

15. The method according to claim 13 or claim 14, wherein said printed marking (7) is provided on said first side (2a) of said backing layer (2), and wherein said step of providing said printed marking (7) is performed after said step b) of arranging an absorbent pad (4) on said first side (2a) of said backing layer (2).

16. The method according to claim 13 or claim 14, wherein said printed marking (7) is provided on the first side (3a) of said adhesive skin-contact layer (3), and wherein said step of providing said printed marking (7) is performed prior to said step c) of arranging said adhesive skin-contact layer on said absorbent pad (4).

## Patentansprüche

1. Medizinischer Verband (1), der eine durchsichtige oder durchscheinende Trägerschicht (2), eine haftende Hautkontaktschicht (3) und ein absorbierendes Kissen (4) umfasst, das zwischen der Trägerschicht (2) und der haftenden Hautkontaktschicht (3) angeordnet ist, wobei das absorbierende Kissen (4) durch umlaufende Ränder (4a-d) definiert ist; wobei die Trägerschicht (2) und die haftende Hautkontaktschicht (3) so angeordnet sind, dass sie sich über die umlaufenden Ränder (4a-d) hinaus erstrecken und damit einen Begrenzungsabschnitt (5) bilden, der das absorbierende Kissen (4) umgibt, wobei die haftende Hautkontaktschicht (3) eine Vielzahl von Löchern (6) in dem Bereich umfasst, der unter dem absorbierenden Kissen (4) liegt, **dadurch gekennzeichnet, dass** der medizinische Verband (1) mindestens eine aufgedruckte Markierung (7) umfasst, die zwischen der Trägerschicht (2) und der haftenden Hautkontaktschicht (3) in dem Begrenzungsabschnitt (5) angeordnet ist, und wobei die haftende Hautkontaktschicht (3) in dem Bereich, der unter der bzw. den aufgedruckten Markierung(en) (7) liegt, keine Löcher aufweist.

2. Medizinischer Verband (1) nach Anspruch 1, wobei die haftende Hautkontaktschicht (3) in dem Bereich, der den Begrenzungsabschnitt (5) bildet, keine Löcher aufweist.

3. Medizinischer Verband (1) nach Anspruch 1 oder Anspruch 2, wobei die Trägerschicht (2) eine erste Seite (2a), die der haftenden Hautkontaktschicht (3) zugewandt ist, und eine gegenüberliegende zweite Seite (2b) aufweist, wobei die mindestens eine aufgedruckte Markierung (7) auf der ersten Seite (2a) der Trägerschicht (2) angeordnet ist.

4. Medizinischer Verband (1) nach einem der Ansprüche 1-3, wobei die haftende Hautkontaktschicht (3) eine erste Seite (3a), die der Trägerschicht (2) zugewandt ist, und eine gegenüberliegende zweite Seite (3b) aufweist, wobei die mindestens eine aufgedruckte Markierung (7) auf der ersten Seite (3a) der haftenden Hautkontaktschicht angeordnet ist.

5. Medizinischer Verband (1) nach einem der vorhergehenden Ansprüche, wobei die haftende Hautkontaktschicht (3) eine Polymerfolie und ein haftendes Silikongel umfasst; wobei das Silikongel so angeordnet ist, dass es im Gebrauch die Haut eines Trägers berührt.

6. Medizinischer Verband (1) nach einem der vorhergehenden Ansprüche, wobei die haftende Hautkontaktschicht (3) durchscheinend oder durchsichtig ist.

7. Medizinischer Verband (1) nach einem der vorhergehenden Ansprüche, wobei die mindestens eine aufgedruckte Markierung (7) mit einer farbigen Tinte hergestellt ist.

8. Medizinischer Verband (1) nach Anspruch 7, wobei der medizinische Verband (1) in dem Bereich des medizinischen Verbands (1), in dem das absorbierende Kissen (4) angeordnet ist, keine(n) aufgedruckte(n) Markierung(en) aufweist.

9. Medizinischer Verband (1) nach einem der vorhergehenden Ansprüche, wobei die mindestens eine aufgedruckte Markierung (7) ein grafisches Element und/oder ein Textelement ist.

10. Medizinischer Verband (1) nach einem der vorhergehenden Ansprüche, wobei die mindestens eine aufgedruckte Markierung (7) eine Chargennummer, eine Produktionslosnummer oder eine Länderangabe ist.

11. Medizinischer Verband (1) nach einem der vorhergehenden Ansprüche, wobei die mindestens eine aufgedruckte Markierung (7) ein maschinenlesbares Kennzeichen, vorzugsweise ein Strichcode oder ein QR-Code ist.

12. Verpackung, vorzugsweise ein Beutel, die den medizinischen Verband (1) nach einem der Ansprüche 1-11 umfasst, wobei die Verpackung mindestens eine aufgedruckte Markierung umfasst, die der mindestens einen aufgedruckten Markierung (7) auf dem medizinischen Verband (1) entspricht.

13. Verfahren zum Herstellen eines medizinischen Verbands, umfassend:
a) Bereitstellen einer durchsichtigen oder durchscheinenden Trägerschicht (2), die eine erste Seite (2a) und eine gegenüberliegende zweite Seite (2b) aufweist;
b) Anordnen eines absorbierenden Kissens (4) auf der ersten Seite (2a) der Trägerschicht (2); wobei das absorbierende Kissen (4) durch umlaufende Ränder (4a-d) definiert ist;
c) Anordnen einer haftenden Hautkontaktschicht (3) auf dem absorbierenden Kissen (4);
wobei die haftende Hautkontaktschicht eine erste Seite (3a), die der Trägerschicht (2) zugewandt ist, und eine gegenüberliegende zweite Seite (3b) aufweist, wobei die Trägerschicht (2) und die haftende Hautkontaktschicht (3) so angeordnet sind, dass sie sich über die umlaufenden Ränder (4a-d) des absorbierenden Kissens (4) hinaus erstrecken und damit einen Begrenzungsabschnitt (5) bilden, der das absorbierende Kissen (4) umgibt, und wobei die haftende Hautkontaktschicht (3) eine Vielzahl von Löchern (6) in dem Bereich umfasst, der unter dem absorbierenden Kissen (4) liegt, **dadurch gekennzeichnet, dass** das Verfahren ferner einen Schritt zum Bereitstellen von mindestens einer aufgedruckten Markierung (7) zwischen der Trägerschicht (2) und der haftenden Hautkontaktschicht (3) in dem Begrenzungsabschnitt (5) umfasst, und wobei die haftende Hautkontaktschicht (3) in dem Bereich, der unter der bzw. den aufgedruckten Markierung(en) (7) liegt, keine Löcher aufweist.

14. Verfahren nach Anspruch 13, wobei die aufgedruckte Markierung (7) auf der ersten Seite (2a) der Trägerschicht (2) oder auf der ersten Seite (3a) der haftenden Hautkontaktschicht (3) bereitgestellt wird.

15. Verfahren nach Anspruch 13 oder Anspruch 14, wobei die aufgedruckte Markierung (7) auf der ersten Seite (2a) der Trägerschicht (2) bereitgestellt wird, und wobei der Schritt zum Bereitstellen der aufgedruckten Markierung (7) nach Schritt b) zum Anordnen eines absorbierenden Kissens (4) auf der ersten Seite (2a) der Trägerschicht (2) durchgeführt wird.

16. Verfahren nach Anspruch 13 oder Anspruch 14, wobei die aufgedruckte Markierung (7) auf der ersten Seite (3a) der haftenden Hautkontaktschicht (3) bereitgestellt wird, und wobei der Schritt zum Bereitstellen der aufgedruckten Markierung (7) vor Schritt c) zum Anordnen der haftenden Hautkontaktschicht auf dem absorbierenden Kissen (4) durchgeführt wird.

## Revendications

1. Pansement médical (1) comprenant une couche support transparente ou translucide (2), une couche adhésive en contact avec la peau (3) et un tampon absorbant (4) disposé entre ladite couche support (2) et ladite couche adhésive en contact avec la peau (3), dans lequel ledit tampon absorbant (4) est défini par des bords périphériques (4a-d) ; ladite couche support (2) et ladite couche adhésive en contact avec la peau (3) étant disposées de manière à s'étendre au-delà desdits bords périphériques (4a-d) pour former une partie de bordure (5) entourant ledit tampon absorbant, ladite couche adhésive en contact avec la peau (3) comprenant une pluralité de perforations (6) dans la zone sous-jacente audit tampon absorbant (4), **caractérisé en ce que** ledit pansement médical (1) comprend au moins un marquage imprimé (7) disposé entre ladite couche support (2) et ladite couche adhésive en contact avec la peau (3) dans ladite partie de bordure (5) et que ladite couche adhésive en contact avec la peau (3) n'est pas perforée dans la zone sous-jacente à la ladite ou auxdites marques imprimées (7).

2. Pansement médical (1) selon la revendication 1, dans lequel ladite couche adhésive en contact avec la peau (3) n'est pas perforée dans la zone formant ladite partie de bordure (5).

3. Pansement médical (1) selon la revendication 1 ou la revendication 2, dans lequel ladite couche support (2) a un premier côté (2a) faisant face à ladite couche adhésive en contact avec la peau (3), et un second côté opposé (2b), ledit au moins un marquage imprimé (7) étant disposé sur ledit premier côté (2a) de ladite couche support (2).

4. Pansement médical (1) selon l'une quelconque des revendications 1 à 3, dans lequel ladite couche adhésive en contact avec la peau (3) a un premier côté (3a) faisant face à ladite couche support (2), et un second côté opposé (3b), ledit au moins un marquage imprimé (7) étant disposé sur ledit premier côté (3a) de ladite couche adhésive en contact avec la peau.

5. Pansement médical (1) selon l'une quelconque des revendications précédentes, dans lequel ladite couche adhésive en contact avec la peau (3) comprend un film polymère et un gel de silicone adhésif ; ledit gel de silicone étant agencé pour entrer en contact avec la peau d'un porteur pendant l'utilisation.

6. Pansement médical (1) selon l'une quelconque des revendications précédentes, dans lequel ladite couche adhésive en contact avec la peau (3) est translucide ou transparente.

7. Pansement médical (1) selon l'une quelconque des revendications précédentes, dans lequel ledit au moins un marquage imprimé (7) est formé par une encre colorée.

8. Pansement médical (1) selon la revendication 7, dans lequel ledit pansement médical (1) est dépourvu de ladite ou desdites marques imprimées dans la zone du pansement médical (1) où ledit tampon absorbant (4) est disposé.

9. Pansement médical (1) selon l'une quelconque des revendications précédentes, dans lequel ledit au moins un marquage imprimé (7) est un élément graphique et/ou un élément de texte.

10. Pansement médical (1) selon l'une quelconque des revendications précédentes, dans lequel ledit au moins un marquage imprimé (7) est un numéro de lot, un numéro de lot de production ou une indication de pays.

11. Pansement médical (1) selon l'une quelconque des revendications précédentes, dans lequel ledit au moins un marquage imprimé (7) est une étiquette lisible par machine, de préférence un code-barres ou un code QR.

12. Emballage, de préférence poche, comprenant le pansement médical (1) selon l'une quelconque des revendications 1 à 11, dans lequel ledit emballage comprend au moins une marque imprimée correspondant à ladite au moins une dite marque imprimée (7) sur ledit pansement médical (1) .

13. Procédé de fabrication d'un pansement médical comprenant les étapes consistant à :
a) prévoir une couche support transparente ou translucide (2) ayant un premier côté (2a) et un second côté opposé (2b) ;
b) disposer un tampon absorbant (4) sur ledit premier côté (2a) de ladite couche support (2) ; ledit tampon absorbant (4) étant défini par des bords périphériques (4a-d) ;
c) disposer une couche adhésive en contact avec la peau (3) sur ledit tampon absorbant (4) ;
ladite couche adhésive en contact avec la peau ayant un premier côté (3a) faisant face à ladite couche support (2), et un second côté opposé (3b), ladite couche support (2) et ladite couche adhésive en contact avec la peau (3) étant disposées de manière à s'étendre au-delà desdits bords périphériques (4a-d) dudit tampon absorbant (4) pour former une partie de bordure (5) entourant ledit tampon absorbant (4), et ladite couche adhésive en contact avec la peau (3) comprenant une pluralité de perforations (6) dans la zone sous-jacente audit tampon absorbant (4), **caractérisé en ce que** ledit procédé comprend en outre une étape consistant à prévoir au moins un marquage imprimé (7) entre ladite couche support (2) et ladite couche adhésive en contact avec la peau (3) dans ladite partie de bordure (5), et que ladite couche adhésive en contact avec la peau (3) n'est pas perforée dans la zone sous-jacente audit ou auxdits marquage(s) imprimé(s) (7).

14. Procédé selon la revendication 13, dans lequel ledit marquage imprimé (7) est prévu sur la première face (2a) de ladite couche support (2) ou sur la première face (3a) de ladite couche adhésive en contact avec la peau (3).

15. Procédé selon la revendication 13 ou la revendication 14, dans lequel ledit marquage imprimé (7) est prévu sur ledit premier côté (2a) de ladite couche support (2), et ladite étape consistant à prévoir ledit marquage imprimé (7) est effectuée après ladite étape b) consistant à disposer un tampon absorbant (4) sur ledit premier côté (2a) de ladite couche support (2).

16. Procédé selon la revendication 13 ou la revendication 14, dans lequel ledit marquage imprimé (7) est prévu sur le premier côté (3a) de ladite couche adhésive en contact avec la peau (3), et ladite étape consistant à prévoir ledit marquage imprimé (7) est effectuée avant ladite étape c) consistant à disposer ladite couche adhésive en contact avec la peau sur ledit tampon absorbant (4).
